(19) **Europäisches Patentamt / European Patent Office / Office européen des brevets**

(11) **EP 1 581 275 B1**

(12) **EUROPEAN PATENT SPECIFICATION**

(45) Date of publication and mention
of the grant of the patent:
**15.03.2017 Bulletin 2017/11**

(21) Application number: **03813798.0**

(22) Date of filing: **18.12.2003**

(51) Int Cl.:
***A61L 27/34*** *(2006.01)*

(86) International application number:
**PCT/US2003/040649**

(87) International publication number:
**WO 2004/056406 (08.07.2004 Gazette 2004/28)**

(54) **BIOMEDICAL DEVICES WITH HYDROPHILIC COATINGS**

BIOMEDIZINISCHE VORRICHTUNG MIT FEUCHTHALTEMITTELBESCHICHTUNGEN

DISPOSITIFS BIOMEDICAUX A REVETEMENTS HYDROPHILES

(84) Designated Contracting States:
**DE FR GB IE IT**

(30) Priority: **19.12.2002 US 325231**

(43) Date of publication of application:
**05.10.2005 Bulletin 2005/40**

(73) Proprietor: **Johnson & Johnson Vision Care, Inc.
Jacksonville, FL 32267 (US)**

(72) Inventors:
• **ZANINI, Diana
Jacksonville, FL 32277 (US)**

• **HEPTING, Joseph, R.
Jacksonville, FL 32257 (US)**

(74) Representative: **Kirsch, Susan Edith et al
Carpmaels & Ransford LLP
One Southampton Row
London WC1B 5HA (GB)**

(56) References cited:
**US-A- 5 002 582    US-A- 5 039 459
US-A- 5 741 551    US-A- 6 087 415**

**Description**

FIELD OF THE INVENTION

This invention relates to coated ophthalmic lenses and a method for coating ophthalmic lenses. In particular, the invention provides ophthalmic lenses on the surfaces of which stable, hydrophilic and/or antimicrobial coatings are formed via reaction of nucleophilic moieties of said coatings with latent carboxylic acid groups present in the ophthalmic lens surface thereby forming ester and/or amide linkages.

BACKGROUND OF THE INVENTION

Devices for use in and on the human body are well known. The chemical composition of the surfaces of such devices plays a pivotal role in dictating the overall efficacy of the devices. Coatings have been used to enhance desirable properties in these devices. In one example, many devices, including catheters, stents, lenses, and implants require biologically non-fouling surfaces, meaning that proteins, lipids, and cells will not adhere to the surface. Coatings could impart these features to the medical devices.

Additionally, these devices, contact lenses in particular, should also be wettable by body fluids in order to ensure wearer comfort. In a further example, coating such devices with an antimicrobial surface, may reduce infections associated with microbes, and would be advantageous.

US 5,741,551 concerns polymeric surfaces with covalently bound polymer molecules.
US 5,002,582 concerns the preparation of polymeric surfaces via covalently attaching polymers.
US 6,087,415 concerns biomedical devices with carboxyl containing hydrophilic coatings.

A wide variety of methods have been developed to coat device surfaces to provide them with the desired characteristics. However, the need still exists for a simple, efficient process that will provide stable coatings.

DETAILED DESCRIPTION OF THE INVENTION AND PREFERRED EMBODIMENTS

The present invention provides a simple, economical process for producing ophthalmic lenses with stable surface coatings. A wide variety of coating types may be applied, such as hydrophilic, antimicrobial, and bioactive coatings, and combinations thereof and the like. By "antimicrobial" what is meant is that bacterial adherence to the device surface is reduced in comparison to the uncoated surface, by 30 percent or more. By "hydrophilic" what is meant is a direct contact angle (advancing) of less than 80°. By "bioactive" what is meant is that the surface provides a beneficial property to the surrounding environment during use. Suitable bioactives, in particular for contact lenses, include anti-histamines, ophthalmic medications, and the like.

Unless otherwise specified, the term weight % is based upon the weight of all components present.

The present invention provides a process for manufacturing a coated ophthalmic lens comprising the steps of: curing a reactive monomer mix to form an ophthalmic lens, the reactive monomer mix comprising at least one latent reactive component in an amount effective to provide sufficient bonding sites for reaction with a coating composition, wherein the latent reactive component is at least one ester compound of the formula R-CO-L, wherein R comprises a group capable of cationic, anionic or free radical polymerisation and L is a leaving group, and reacting the ophthalmic lens with a coating composition in an amount sufficient to coat the surface of the ophthalmic lens, the coating composition comprising one or more nucleophilic moieties selected from the group consisting of alcohol, primary amine, secondary amine, thiol and combinations thereof to form the coated ophthalmic lens.

The present invention further provides an ophthalmic lens formed from curing a reactive monomer mix comprising at least one latent reactive component in an amount effective to provide sufficient bonding sites for reaction with a coating composition, wherein the latent reactive component is at least one ester compound of the formula R-CO-L, wherein R comprises a group capable of cationic, anionic or free radical polymerisation and L is a leaving group.

In a preferred embodiment, the ophthalmic lens is a contact lens.

It is an unexpected discovery of the invention that a carboxylate functionality may be readily incorporated into a variety of polymeric articles and subsequently reacted with nucleophilic coating compositions to form articles with desirable properties. The method of the present invention provides a convenient way to covalently bond a variety of coatings to formed polymeric articles. The coatings of the present invention are stable, as well as providing the desired property enhancements. By "stable" is meant that subjecting the coating to autoclaving, washing with a cleaning agent, and/or rinsing with a saline solution does not substantially alter the chemical properties of the biomedical device or coating.

Latent reactive components used in the invention are ester compounds of the formula R-CO-L wherein R comprises a group capable of cationic, anionic or free radical polymerization and L is a leaving group. Suitable R groups include monovalent groups that can undergo free radical and/or cationic polymerization comprising up to 20 carbon atoms. Preferred R groups comprise free radical reactive groups, such as acrylates, styryls, vinyls, vinyl ethers,

$C_{1-6}$alkylacrylates, acrylamides, $C_{1-6}$alkylacrylamides, N-vinyllactams, N-vinylamides, $C_{2-12}$alkenyls, $C_{2-12}$alkenylphenyls, $C_{2-12}$alkenylnaphthyls, or $C_{2-6}$alkenylphenyl$C_{1-6}$alkyls or a cationic reactive group such as vinyl ethers or epoxide groups and mixtures thereof. Particularly preferred R groups include methacrylates, acryloxys, methacrylamides, acrylamides, and mixtures thereof.

Suitable L groups are stable under reaction conditions, and protect the carboxylate group and leave readily under coating conditions. Suitable L groups include alkyl esters, phenyl esters, hydroxy para-nitroaryls, *p*-nitrophenyl esters, N-hydroxylamine derivatives, and tosylate esters all of which may be substituted or unsubstituted. Preferred L groups include *t*-butyl esters, 2,4,5-trichlorophenyl esters, pentafluorophenyl esters, N-hydroxysuccinimide esters, N-hydroxy-oxo-dihydrobenzotriazine derivatives, 1-hydroxybenzotriazole esters, tosylate esters and combinations thereof. Preferred suitable L groups include pentafluorophenyl esters, tosylate esters, and N-hydroxysuccinimide esters, and mixtures thereof. Preferred latent reactive compounds include pentafluoromethacrylate and N-acryloxysuccinimide and mixtures thereof.

The latent reactive component is included in the monomer mix in a coating effective amount. Any amount sufficient to provide the desired level of bonding sites for the coating polymer is sufficient. Suitable amounts include between 0.01 and 10 weight %, preferably between 0.01 and 5 weight %, and more preferably between 0.01 and 1 weight %, all based upon the total weight of the reactive components, all based upon the weight of all the components in the monomer mix.

The latent reactive component may be added to any lens material, but is particularly useful for lens materials which do not contain carboxylic acid groups. Suitable lens materials include silicone hydrogels. The reactive components which are useful for making silicone hydrogels are known and comprise silicone containing components, hydrophilic components and optionally, fluorine containing components. Suitable silicone containing components include silicone containing monomers, prepolymers, and macromers. Suitable fluorine containing components include fluorine containing monomers, prepolymers, and macromers.

Suitable siloxane containing monomers include 3-methacryloxy-2-hydroxypropyloxy)propyltris(trimethylsiloxy)silane (SiGMA), 3-methacryloxypmpyltris(trimethylsiloxy)silane (TRIS), amide analogs of TRIS described in U.S. 4,711,943, vinylcarbamate or carbonate analogs decribed in U.S. Pat. 5,070,215, and monomers contained in U.S. Pat. 6,020,445. More specifically, 3-methacryloxypropyltris(trimethylsiloxy)silane (TRIS), monomethacryloxypropyl terminated polydimethylsiloxanes, polydimethylsiloxanes, 3-methacryloxypropylbis(trimethylsiloxy)methylsilane, methacryloxypropylpentamethyl disiloxane, and combinations thereof are particularly useful as siloxane containing monomers.

Suitable siloxane containing macromers have a number average molecular weight between 5,000 and 15,000 Daltons. Siloxane containing macromers include materials comprising at least one siloxane group, and preferably at least one dialkyl siloxane group, and more preferably at least one dimethyl siloxane group. The siloxane containing macromers may include other components such as urethane groups, alkylene or alkylene oxide groups, polyoxyalkalene groups, arylene groups, alkyl esters, amide groups, carbamate groups, perfluoroalkoxy groups, isocyanate groups, combinations thereof and the like. A preferred class of siloxane containing macromers may be formed via the polymerization of one or more siloxanes with one or more acrylic or methacrylic materials. Siloxane containing macromers may be formed via group transfer polymerization ("GTP"), free radical polymerization or condensation reactions. The siloxane containing macromers may be formed in one or a series of steps depending on the components selected and using conditions known in the art. Specific siloxane containing macromers, and methods for their manufucture, include those disclosed in US 5,760,100 as materials A-D (methacrylate functionalized, silicone-fluoroether urethanes and methacrylate functionalized, silicone urethanes), and those disclosed in US 6,367,929 (styrene functionalized prepolymers of hydroxyl functional methacrylates and silicone methacrylates).

Suitable siloxane containing reactive prepolymers include vinyl carbamate functionalized polydimethylsiloxane, which is further disclosed in US 5,070215 and urethane based prepolymers comprising alternating "hard" segments formed from the reaction of short chained diols and diisocyantes and "soft" segments formed from a relatively high molecular weight polymer, which is $\alpha,\omega$ endcapped with two active hydrogens. Specific examples of suitable siloxane containing prepolymers, and methods for their manufacture, are disclosed in US 5,034,461.

Generally, the siloxane containing component is present in amounts between 5 and 50 weight %, preferably between 10 and 50 weight %, and more preferably between 15 and 45 weight %, all based upon the total weight of the reactive components.

Suitable fluorine containing monomers include fluorine-containing (meth)acrylates, and more specifically include, for example, fluorine-containing $C_2$-$C_{12}$ alkyl esters of (meth)acrylic acid such as 2,2,2-trifluoroethyl (meth)acrylate, 2,2,2,2',2',2'-hexafluoroisopropyl (meth)acrylate, 2,2,3,3,4,4,4-heptafluorobutyl (meth)acrylate, 2,2,3,3,4,4,5,5,6,6,7,7,8,8,8- pentadecafluorooctyl (meth)acrylate, 2,2,3,3,4,4,5,5,6,6,7,7,8,8,9,9- hexadecafluorononyl (meth)acrylate and the like. Fluorine containing macromers and reactive prepolymers include macromers and prepolymers which include said flurorine containing monomers. Fluorine containing components may be present in amounts from about 0 to 10 weight %.

The reactive components of the present invention may also include any hydrophilic monomers used to prepare conventional hydrogels. For example monomers containing acrylic groups ($CH_2$=CRCOX, where R is hydrogen or $C_{1-6}$alkyl an X is O or N) or vinyl groups (-C=$CH_2$) may be used. Examples of additional hydrophilic monomers are N,N-dimethylacrylamide, 2-hydroxyethyl methacrylate, glycerol monomethacrylate, 2-hydroxyethyl methacrylamide, polyethyleneglycol monomethacrylate, methacrylic acid, acrylic acid, N-vinyl pyrrolidone, N-vinyl-N-methyl acetamide, N-vinyl-N-ethyl acetamide, N-vinyl-N-ethyl formamide, N-vinyl formamide and combinations thereof.

Aside from the hydrophilic monomers mentioned above, polyoxyethylene polyols having one or more of the terminal hydroxyl groups replaced with a functional group containing a polymerizable double bond may be used. Examples include polyethylene glycol, as disclosed in US 5,484,863, ethoxylated alkyl glucoside, as disclosed in US 5,690,953, US 5,304,584, and ethoxylated bisphenol A, as disclosed in US 5,565,539, reacted with one or more molar equivalents of an end-capping group such as isocyanatoethyl methacrylate, methacrylic anhydride, methacryloyl chloride, vinylbenzoyl chloride, and the like, produce a polyethylene polyol having one or more terminal polymerizable olefinic groups bonded to the polyethylene polyol through linking moieties such as carbamate, urea or ester groups.

Still further examples include the hydrophilic vinyl carbonate or vinyl carbamate monomers disclosed in U.S. Pat. Nos. 5,070,215, the hydrophilic oxazolone monomers disclosed in U.S. Pat. No. 4,910,277, and polydextran.

The preferred additional hydrophilic monomers are N,N-dimethylacrylamide (DMA), 2-hydroxyethyl methacrylate (HEMA), glycerol methacrylate, 2-hydroxyethyl methacrylamide, N-vinylpyrrolidone (NVP), polyethyleneglycol monomethacrylate, and combinations thereof, with hydrophilic monomers comprising DMA being particularly preferred. Additional hydrophilic monomers may be present in amounts of 0 to 70 weight %, more preferably of 5 and 60 weight %, and most preferably of 10 and 50 weight %, based upon the total weight of the reactive components.

The reactive components may also comprise additional components such as crosslinkers, photoinitiators, UV absorbing compounds and monomers, visibility tinting agents, reactive tints, antimicrobial compounds, release agents, pigments and dyes, photochromic compounds, combinations thereof and the like. The reactive components are mixed together in the presence of a diluent to form a reaction mixture. Suitable diluents are disclosed in US 6,020,455.

Suitable lens materials include aquafilcon A, balafilcon A, lotrafilcon A, and the like.

Various processes are known for molding the reaction mixture in the production of contact lenses, including spincasting and static casting. Spincasting methods are disclosed in U.S. Patents Nos. 3,408,429 and 3,660,545, and static casting methods are disclosed in U.S. Patents Nos. 4,113,224 and 4,197,266. The preferred method for producing contact lenses comprising the polymer of this invention is by the direct molding of the silicone hydrogel, which is economical, and enables precise control over the final shape of the hydrated lens. For this method, the reaction mixture is placed in a mold having the shape of the final desired silicone hydrogel, i.e. water-swollen polymer, and the reaction mixture is subjected to conditions whereby the monomers polymerize, to thereby produce a polymer in the approximate shape of the final desired product Then, this polymer mixture is optionally treated with a solvent and then water, producing a silicone hydrogel having a final size and shape which are quite similar to the size and shape of the original molded polymer article. This method can be used to form contact lenses and is further described in U.S. Patents Nos. 4,495,313; 4,680,336; 4,889,664; and 5,039,459.

After the biomedical device has been formed, it is reacted with a coating compound or polymer. Any compound (molecule and/or polymer) which is capable of reacting with a carboxylate to form an ester or an amide may be used for the coating polymer. Suitable coating compounds or polymers contain one or more nucleophilic moieties such as alcohols, primary and secondary amines, and thiol functionalities. These coating compounds include molecules, or polymers that contain these functionalities, mixtures thereof and the like. Suitable coating compounds and polymers include vitamins, anti-histamines, antibacterials, UV blockers, dyes and tints, biodegradable polymers, polyols, polyamines, anti-microbials, wetting agents, metal chelators, lachrymators, pro-drugs, peptidoglycans, oligosaccharides, polysaccharides, aminoglycosides, glycopeptides, combinations thereof and the like. Specific examples of coating compounds include polyHEMA (poly(2-hydroxyethyl methacrylate), pHEMA), β-lactam antibiotics functionalized with either an amino group or a hydroxyl group, penicillins functionalized with either an amino group or a hydroxyl group, phenylglycine, 4-hydroxyphenylgycine, cephalosporins functionalized with either an amino group or a hydroxyl group, cephaloglycine, cephalexin, cephadroxil, carbapenems functionalized with either an amino group or a hydroxyl group, streptomycin, gentomicin, amikacin, oxazolidinones functionalized with either an amino group or a hydroxyl group, tetracyclines functionalized with either an amino group or a hydroxyl group, glycylcyclines functionalized with either an amino group or a hydroxyl group, quinolones functionalized with either an amino group or a hydroxyl group, fluoroquinolones functionalized with either an amino group or a hydroxyl group, macrolides functionalized with either an amino group or a hydroxyl group, ketolides functionalized with either an amino group or a hydroxyl group, streptogramins functionalized with either an amino group or a hydroxyl group, vancomycin derivatives functionalized with either an amino group or a hydroxyl group, teicoplanin derivatives functionalized with either an amino group or a hydroxyl group, avoparcin derivatives functionalized with either an amino group or a hydroxyl group, combinations thereof and the like. Preferred classes of coating compounds and polymers include wetting agents, antimicrobials, UV blockers, antibacterials, biodegradable polymers, combinations thereof and the like.

Preferred coating polymers include polyalcohols, polyamines, bioactive compounds that are known to have amine and/or alcohol funcitonalities and mixtures thereof. Examples of suitable coating polymers include polyHEMA.

The coating compound or polymer may have any molecular weight. Generally, coating polymers have molecular weights between 100 and 1,000,000, preferably between 1,000 and 500,000 $M_v$ Molecular weights can be measured in a variety of ways including, but not limited to, molecular mass spectrometry and size exclusion methods such as gel filtration chromatography and gel permeation chromatography.

In the process of the invention, the surface to be coated is contacted with the coating polymer in any convenient manner. For example, the device may be placed in a solution of coating polymer and solvent and coupling additives.

Suitable solvents for use in the invention are non-nucleophilic solvents capable of solubilizing the coating polymer without negatively reacting with the biomedical device. Suitable solvents include, but are not limited to, DMF, DMSO, methylene chloride, ethyl acetate, DPMA, mixtures thereof and the like. Preferred solvents include DMF and DPMA.

The device is contacted with the solvent/coating polymer solution under conditions suitable to form the coating. Suitable temperatures include those between the freezing and boiling points of the selected solvent, preferably between 0 and 100°C and more preferably between 20 and 50 °C. The contact time used will be a length of time sufficient to coat the surface to the extent desired. Contact times may be up to 2 days, preferably up to 1 day, and most preferably up to 12 hours. Pressure is not critical in the coating reaction of the present invention. However, those of skill in the art will recognize that elevated pressures and temperatures will enable the reaction to be conducted in a shorter period of time.

Coupling additives are any compound(s) that enables the amide and/or ester linkage between the device(s) and coating(s) to be formed more readily than without their addition and include, but are not limited to, trans-esterification reagents, catalysts, thereof and the like. Examples include 4-dimethylaminopyridine (DMAP), 1-(3-dimethylaminopropyl)-3-ethylcarbodiimide hydrochloride salt (EDC), 1,3-diisopropylcarbodiimide, 1,3-dicyclohexylcarbodiimide, 1-hydroxybenzotriazole (HOBt), 1-hydroxybenzotriazole hydrate, crown ethers, acids, bases, enzymes, combinations thereof and the like.

A coating effective amount of coating polymer is used, meaning an amount sufficient to coat the surface to the desired degree. Generally, the amount of coating compound or polymer used is 0.1 to 20 weight %, preferably 0.5 to 10 wieght %, and more preferably, 0.8 to 5 weight % of the coating solution.

Following contacting, the surface may be washed with water or buffered saline solution to remove unrelated (or unreacted) polymer, leaving group, solvent, and byproducts. Optionally, the coated surface may be heated in water to extract residual coating, leaving group, and byproducts and to ensure the break down of leaving group complexes that may have formed.

The invention will be further clarified by a consideration of the following, nonlimiting examples. The following tests were used in the examples.

Lenses were analyzed for their coatings using the FTIR-ATR line scan technique using a Perkin-Elmer Spectrum GX FTIR AutoIMAGE System. All line scans were made with 300-micron incremental steps from edge to edge in the center region of the lens. All samples were analyzed in wet state.

The advancing contact angle was measured as follows. At least three samples from. each set were prepared by cutting out a center strip from the lens approximately 5 mm in width and equilibrated in packing solution. The wetting force between the lens surface and borate buffered saline is measured at 23°C using a Wilhelmy microbalance while the sample is being immersed into or pulled out of the saline. The following equation is used

$$F = 2\gamma p\cos\theta \qquad \text{or} \qquad \theta = \cos^{-1}(F/2\gamma p)$$

where F is the wetting force, $\gamma$ is the surface tension of the probe liquid, p is the perimeter of the sample at the meniscus and $\theta$ is the contact angle. The advancing contact angle is obtained from the portion of the wetting experiment where the sample is being immersed into the packing solution. Each sample was cycled four times and the results were averaged to obtain the advancing contact angles for the lens.

Haze is measured by placing a hydrated test lens in borate buffered saline in a clear 20 x 40 x 10 mm glass cell at ambient temperature above a flat black background, illuminating from below with a fiber optic lamp (Titna Tool Supply Co. fiber optic light with 0.5" diameter light guide set at a power setting of 4-5.4) at an angle 66° normal to the lens cell, and capturing an image of the lens from above, normal to the lens cell with a video camera (DVC 1300C:19130 RGB camera with Navitar TV Zoom 7000 zoom lens) placed 14 mm above the lens platform. The background scatter is subtracted from the scatter of the lens by subtracting an image of a blank cell using EPIX XCAP V 1.0 software. The subtracted scattered light image is quantitatively analyzed, by integrating over the central 10 mm of the lens, and then comparing to a -1.00 diopter CSI Thin Lens®, which is arbitrarily set at a haze value of 100, with no lens set as a haze value of 0. Five lenses are analyzed and the results are averaged to generate a haze value as a percentage of the standard CSI lens.

The water content was measured as follows: lenses to be tested are allowed to sit in packing solution for 24 hours. Each of three test lens are removed from packing solution using a sponge tipped swab and placed on blotting wipes which have been dampened with packing solution. Both sides of the lens are contacted with the wipe. Using tweezers, the test lens are placed in a weighing pan and weighed. Two more sets of samples are prepared and weighed as above. The pan is weighed three times and the average is the wet weight.

The dry weight is measured by placing the sample pans in a vacuum oven which has been preheated to 60°C for 30 minutes. Vacuum is applied until at least 0.4 inches Hg is attained. The vacuum valve and pump are turned off and the lenses are dried for four hours. The purge valve is opened and the oven is allowed reach atmospheric pressure. The pans are removed and weighed. The water content is calculated as follows:

$$\text{Wet weight} = \text{combined wet weight of pan and lenses} - \text{weight of weighing pan}$$

$$\text{Dry weight} = \text{combined dry weight of pan and lens} - \text{weight of weighing pan}$$

$$\% \text{ water content} = \frac{(\text{wet weight} - \text{dry weight})}{\text{wet weight}} \times 100$$

The average and standard deviation of the water content are calculated for the samples are reported.

Modulus is measured by using the crosshead of a constant rate of movement type tensile testing machine equipped with a load cell that is lowered to the initial gauge height. A suitable testing machine includes an Instron model 1122. A dog-bone shaped sample having a 0.522 inch length, 0.276 inch "ear" width and 0.213 inch "neck" width is loaded into the grips and elongated at a constant rate of strain of 2 in/min. until it breaks. The initial gauge length of the sample (Lo) and sample length at break (Lf) are measured. Twelve specimens of each composition are measured and the average is reported. Tensile modulus is measured at the initial linear portion of the stress/strain curve.

Examples

Example 1

To a dry container housed in a dry box under nitrogen at ambient temperature was added 30.0 g (0.277 mol) of bis(dimethylamino)methylsilane, a solution of 13.75 ml of a 1M solution of tetrabutyl ammonium-m-chlorobenzoate (TBACB) (386.0 g TBACB in 1000 ml dry THF), 61.39 g (0.578 mol) of p-xylene, 154.28 g (1.541 mol) methyl methacrylate (1.4 equivalents relative to initiator), 1892.13 g (9.352 mol) 2-(trimethylsiloxy)ethyl methacrylate (8.5 equivalents relative to initiator) and 4399.78 g (61.01 mol) of THF. To a dry, three-necked, round-bottomed flask equipped with a thermocouple and condenser, all connected to a nitrogen source, was charged the above mixture prepared in the dry box.

The reaction mixture was cooled to 15 °C while stirring and purging with nitrogen. After the solution reaches 15 °C, 191.75 g (1.100 mol) of 1-trimethylsiloxy-1-methoxy-2-methylpropene (1 equivalent) was injected into the reaction vessel. The reaction was allowed to exotherm to approximately 62 °C and then 30 ml of a 0.40 M solution of 154.4 g TBACB in 11 ml of dry THF was metered in throughout the remainder of the reaction. After the temperature of reaction reached 30 °C and the metering began, a solution of 467.56 g (2.311 mol) 2-(trimethylsiloxy)ethyl methacrylate (2.1 equivalents relative to the initiator), 3636.6. g (3.463 mol) n-butyl monomethacryloxypropyl-polydimethylsiloxane (3.2 equivalents relative to the initiator), 3673.84 g (8.689 mol) TRIS (7.9 equivalents relative to the initiator) and 20.0 g bis(dimethylamino)methylsilane was added.

The mixture was allowed to exotherm to approximately 38-42 °C and then allowed to cool to 30 °C. At that time, a solution of 10.0 g (0.076 mol) bis(dimethylamino)methylsilane, 154.26 g (1.541 mol) methyl methacrylate (1.4 equivalents relative to the initiator) and 1892.13 g (9.352 mol) 2-trimethylsiloxy)ethyl methacrylate (8.5 equivalents relative to the initiator) was added and the mixture again allowed to exotherm to approximately 40 °C. The reaction temperature dropped to approximately 30 °C and 2 gallons of THF were added to decrease the viscosity. A solution of 439.69 g water, 740.6 g methanol and 8.8 g (0.068 mol) dichloroacetic acid was added and the mixture refluxed for 4.5 hours to de-block the protecting groups on the HEMA. Volatiles were then removed and toluene added to aid in removal of the water until a vapor temperature of 110 °C was reached.

The reaction flask was maintained at approximately 110 °C and a solution of 443 g (2.201 mol) TMI and 5.7 g (0.010 mol) dibutyltin dilaurate were added. The mixture was reacted until the isocyanate peak was gone by IR. The toluene was evaporated under reduced pressure to yield an off-white, anhydrous, waxy reactive monomer. The macromer was placed into acetone at a weight basis of approximately 2:1 acetone to macromer. After 24 hrs, water was added to precipitate out the macromer and the macromer was filtered and dried using a vacuum oven between 45 and 60 °C for 20-30 hrs.

Examples 2 - 8

A reaction mixture was formed by adding 100 parts of the components shown in Table 1, in the amounts shown in Table 1 with 20 parts 3,7-dimethyl-3-octanol. Specifically, in the following order macromer, Norbloc 7966, diluent, TEGDMA, HEMA, DMA, TRIS, and mPDMS were added to an amber flask. These components were mixed at 170-300 rpm, at 50-55 °C, for 90 to 180 minutes. While maintaining mixing, blue HEMA was added and the components mixed for a further 20 to 75 minutes (at 170-300 rpm, 50-55 °C). Still with mixing, PVP was added and the mixture stirred for another 20 to 140 minutes (at 170-300 rpm, 50-55 °C). Lastly, with continual mixing, CGI 1850 (Irgacure 1850) was added.

Table 1

| Component | Weight Percent |
|---|---|
| Macromer (Ex 1) | 18.95 |
| TRIS | 14.74 |
| DMA | 27.37 |
| MPDMS | 29.47 |
| NORBLOC | 2.11 |
| CGI 1850 | 1.05 |
| TEGDMA | 1.05 |
| HEMA | 5.26 |

Pentafluorophenyl methacrylate (OPfp) or N-acryloxysuccinimide (NAS) was added to the reaction mixture in the amounts shown in Table 2, below. The resulting mixtures were mixed vigorously for approximately 10 minutes (or until the solution appeared clear and evenly mixed) and the then degassed, on high vacuum, until no air bubbles were visible in the reaction mixture (about 20 minutes). The reaction mixtures were placed into thermoplastic contact lens molds, and irradiated using Philips TL 20W/03T fluorescent bulbs at 50°C, for about 50 minutes in an $N_2$ atmosphere. The lenses containing OPfp or NAS were demolded in Dowanol® DPMA (commercially available from Aldrich) and washed up to five times in DPMA. Each wash lasted about 120 minutes. Lenses were then placed in N,N-dimethylformamide (DMF) (1 lens/2 mL) containing between 0.50 and 10 v/v of N, N-diisopropylethylamine (DIPEA) and between 2.0 and 5.0 weight percent of polyHEMA (MW 300,000) and stirred overnight at 25° C. The amounts are shown in Table 2, below. The solution was next decanted and the lenses suspended in fresh DMF (1 lens/2 mL). After stirring for about 60 minutes, lenses were washed three times with de-ionized water (DI water) for approximately 60 minutes each time. Lenses were packaged in glass vials containing a minimum of 3 mL packing (saline) solution and autoclaved (30 minutes, 121 °C). The lenses were analyzed to determine carbon to silicon ratio (via FTIR), advancing direct contact angle, haze and modulus. The results are shown in Table 3, below.

Table 2

| Ex. # | latent | wt% latent | V/v solvent | Wt% pHEMA |
|---|---|---|---|---|
| 2 | - | - | - | - |
| 3 | OPfp | 0.5 | 0.62 | 0 |
| 4 | OPfp | 0.5 | 0.62 | 2 |
| 5 | OPfp | 0.5 | 10 | 5 |
| 6 | NAS | 0.5 | 0.62 | 0 |
| 7 | NAS | 0.5 | 0.62 | 2 |
| 8 | NAS | 0.5 | 10 | 5 |

Table 3

| Ex # | C/Si Ratio* | DMA (°) | Haze (%) |
|------|-------------|---------|----------|
| 2 | 0.62(0.02) | variable | 10 |
| 3 | 0.61(0.09) | 77(14) | - |
| 4 | 1.02(0.02) | 77(9) | 64(4) |
| 5 | 0.87(0.03) | 53(12) | 55(7) |
| 6 | 0.69(0.06) | 94(16) | - |
| 7 | 0.83(0.04) | 75(11) | 50(5) |
| 8 | 0.86(0.01) | 50(3) | 23(0.5) |
| *N $\geq$ 45, standard deviation in parentheses. | | | |

Examples 4, 5, 7 and 8 clearly show an increased carbon:silicon ratio, indicating that polyHEMA was coated on all of the lenses. Thus, according to FTIR-ATR studies, both OPfp and NAS pathways are successful in the covalent attachment of polyHEMA to silicone hydrogel lenses.

The lenses of the present invention also show good wettability and acceptable haze levels.

Examples 9 - 12

Example 5 was repeated except that amounts of OPfp, the temperature used during the coating step, and the lens/solvent ratios in some of the steps were varied. That is, Example 5 was repeated up until pentafluorophenyl methacrylate (OPfp) addition. OPfp was added in the amounts shown in Table 4. The resulting mixtures were mixed vigorously for approximately 10 minutes (or until the solution appeared clear and evenly mixed) and the then degassed, on high vacuum, until no air bubbles were visible in the reaction mixture (about 20 minutes). The reaction mixtures were placed into thermoplastic contact lens molds, and irradiated using Philips TL 20W/03T fluorescent bulbs at 50°C, for about 50 minutes in an $N_2$ atmosphere. The lenses containing OPfp were demolded in Dowanol® DPMA (commercially available from Aldrich) and washed up to five times in DPMA. Each wash lasted about 120 minutes. Lenses were then placed in N,N-dimethylformamide (DMF) (1 lens/3 mL) containing between 0.50 and 10 v/v of N, N-diisopropylethylamine (DIPEA) and between 2.0 and 5.0 weight percent of polyHEMA (MW 300,000) and stirred overnight at the temperatures shown in Table 3. The solution was next decanted and the lenses suspended in fresh DMF (1 lens/30 mL). After stirring for about 60 minutes, lenses were washed up to three times with de-ionized water (DI water) for approximately 60 minutes each time. Lenses were packaged in glass vials containing a minimum of 3 mL packing (saline) solution and autoclaved (30 minutes, 121 °C). The lenses were analyzed to determine advancing direct contact angle and haze. The results are shown in Table 4, below.

Table 4

| Ex. # | % OPfp | Temp (°C) | Adv.CA | Haze |
|-------|--------|-----------|--------|------|
| 5 | 0.5 | 25 | 53(12) | 55(7) |
| 9 | 0.5 | 35 | 81(12) | 16(1) |
| 10 | 1.0 | 35 | 80(5) | 20(2) |
| 11 | 5.0 | 35 | 73(10) | 13(2) |
| 12 | 5.0 | 50 | 69(8) | Nm |
| Nm= not measured | | | | |

The lenses treated at 50°C overnight (Example 12) were fragile and only lens pieces were recovered. All samples show reduced contact angles, indicating that the coating attached to the lens. While the coating temperature used in Example 12 may have been higher than desired for contact lenses, it would be effective for more robust medical devices, such as stents, catheters and the like. Therefore, a wide range of coating concentrations and temperatures may be attached via the process of the present invention.

Examples 13 - 16

Example 8 was repeated except that amounts of NAS, the temperature used during the coating step, and the lens/solvent ratios in some of the steps were varied. That is, Example 8 was repeated up until N-acryloxy succinimide (NAS) addition. NAS was added in the amounts shown in Table 5. The resulting mixtures were mixed vigorously for approximately 10 minutes (or until the solution appeared clear and evenly mixed) and the then degassed, on high vacuum, until no air bubbles were visible in the reaction mixture (about 20 minutes). The reaction mixtures were placed into thermoplastic contact lens molds, and irradiated using Philips TL 20W/03T fluorescent bulbs at 50°C, for about 50 minutes in an $N_2$ atmosphere. The lenses containing NAS were demolded in Dowanol® DPMA (commercially available from Aldrich) and washed up to five times in DPMA. Each wash lasted about 120 minutes. Lenses were then placed in N,N-dimethylformamide (DMF) (1 lens/3 mL) containing between 0.50 and 10 v/v of N, N-diisopropylethylamine (DIPEA) and between 2.0 and 5.0 weight percent of polyHEMA (MW 300,000) and stirred overnight at the temperatures shown in Table 3. The solution was next decanted and the lenses suspended in fresh DMF (1 lens/30 mL). After stirring for about 60 minutes, lenses were washed up to three times with de-ionized water (DI water) for approximately 60 minutes each time. Lenses were packaged in glass vials containing a minimum of 3 mL packing (saline) solution and autoclaved (30 minutes, 121 °C). The lenses were analyzed to determine advancing direct contact angle and haze. The results are shown in Table 5, below.

Table 5

| Ex. # | % NAS | Temp (°C) | Adv.CA | Haze |
|-------|-------|-----------|--------|------|
| 8 | 0.5 | 25 | 50(3) | 23(0.5) |
| 13 | 0.5 | 35 | 87(20) | 35(3) |
| 14 | 1.0 | 35 | 98(9) | 95(7) |
| 15 | 5.0 | 35 | 76(15) | 373(29) |
| 16 | 5.0 | 50 | 81(14) | Nm |
| Nm= not measured | | | | |

The lenses treated at 50°C overnight were fragile and only lens pieces were recovered. All samples show reduced contact angles, indicating that the coating attached to the lens. While the coating temperature used in Example 12 may have been higher than desired for contact lenses, it would be effective for more robust medical devices, such as stents, catheters and the like. A wide range of coating concentrations may be attached via the process of the present invention.

**Claims**

1. A process for manufacturing a coated ophthalmic lens comprising the steps of:

curing a reactive monomer mix to form an ophthalmic lens, the reactive monomer mix comprising at least one latent reactive component in an amount effective to provide sufficient bonding sites for reaction with a coating composition, wherein the latent reactive component is at least one ester compound of the formula R-CO-L, wherein R comprises a group capable of cationic, anionic or free radical polymerisation and L is a leaving group, and
reacting the ophthalmic lens with a coating composition in an amount sufficient to coat the surface of the ophthalmic lens, the coating composition comprising one or more nucleophilic moieties selected from the group consisting of alcohol, primary amine, secondary amine, thiol and combinations thereof to form the coated ophthalmic lens.

2. The process of claim 1 wherein the ophthalmic lens is a contact lens.

3. The process of claim 1 wherein said R group is selected from the group consisting of acrylates, styryls, vinyls, $C_{1-6}$alkylacrylates, acrylamides, $C_{1-6}$alkylacrylamides, N-vinyllactams, N-vinylamides, $C_{2-12}$alkenyls, $C_{2-12}$alkenylphenyls, $C_{2-12}$alkenylnaphthyls, $C_{2-6}$alkenylphenyl$C_{1-6}$alkyls, vinyl ethers and epoxide groups and combinations thereof.

4. The process of claim 1 wherein said R group is selected from the group consisting of methacrylates, acryloxys, acrylamides, methacrylamides and combinations thereof.

5. The process of claim 1 wherein said L group is selected from the group consisting of hydroxyalkyls, hydroxyaryls, hydroxy para-nitroaryls, alkyl esters, phenyl esters, *p*-nitrophenyl esters, N-hydroxylamine derivatives, and tosylates all of which may be substituted or unsubstituted.

6. The process of claim 1 wherein said L group is selected from the group consisting of *t*-butyl esters, 2,4,5-trichlorophenyl esters, pentafluorophenyl esters, N-hydroxysuccinimide esters, N-hydroxy-oxo-dihydrobenzotriazine derivatives, and 1-hydroxybenzotriazole esters.

7. The process of claim 1 wherein said L group is selected from the group consisting of pentafluorophenyl esters and N-hydroxysuccinimide esters

8. The process of claim 1 wherein said at least one latent reactive compound comprises pentafluoromethacrylate, N-acryloxysuccinimide and mixtures thereof.

9. The process of claim 1 wherein said latent reactive component is included in the reactive monomer mix in an amount between 0.01 and 10 weight % based upon the total weight of the reactive components.

10. The process of claim 1 wherein said latent reactive component is included in the reactive monomer mix in an amount between 0.01 and 5 weight % based upon the total weight of the reactive components.

11. The process of claim 1 wherein said latent reactive component is included in the reactive monomer mix in an amount between 0.01 and 1 weight%, based upon the total weight of the reactive components.

12. The process of claim 2 wherein said reactive monomer mix comprises at least one silicone containing component and at least one hydrophilic component.

13. The process of claim 2 wherein said coating compound or polymer is selected from the group consisting of vitamins, anti-histamines, antibacterials, UV blockers, dyes and tints, biodegradable polymers, polyols, polyamines, anti-microbials, wetting agents, metal chelators, lachrymators, pro-drugs, peptidoglycans, oligosaccharides, polysaccharides, aminoglycosides, glycopeptides and combinations thereof.

14. The process of claim 2 wherein said coating compound or polymer is selected from the group consisting of polyHEMA, β-lactam antibiotics functionalized with either an amino group or a hydroxyl group, penicillins functionalized with either an amino group or a hydroxyl group, phenylglycine, 4-hydroxyphenylglycine, cephalosporins functionalized with either an amino group or a hydroxyl group, cephaloglycine, cephalexin, cephadroxil, carbapenems functionalized with either an amino group or a hydroxyl group, streptomycin, gentomicin, amikacin, oxazolidinones functionalized with either an amino group or a hydroxyl group, tetracyclines functionalized with either an amino group or a hydroxyl group, glycylcyclines functionalized with either an amino group or a hydroxyl group, quinolones functionalized with either an amino group or a hydroxyl group, fluoroquinolones functionalized with either an amino group or a hydroxyl group, macrolides functionalized with either an amino group or a hydroxyl group, ketolides functionalized with either an amino group or a hydroxyl group, streptogramins functionalized with either an amino group or a hydroxyl group, vancomycin derivatives functionalized with either an amino group or a hydroxyl group, teicoplanin derivatives functionalized with either an amino group or a hydroxyl group, avoparcin derivatives functionalized with either an amino group or a hydroxyl group and combinations thereof.

15. The process of claim 2 wherein said coating compound or polymer is selected from the group consisting of wetting agents, antimicrobials, UV blockers, antibacterials, biodegradable polymers, combinations thereof.

16. The process of claim 2 wherein said coating compound or polymer is a polymer selected from the group consisting of polyalcohols, polyamines, bioactive compounds comprising amine and/or alcohol functionalities and mixtures thereof.

17. The process of claim 2 wherein said coating compound or polymer comprises polyHEMA, and mixtures thereof.

18. The process of claim 1 wherein said step of reacting the ophthalmic lens with a coating composition comprises placing said ophthalmic lens in a solution comprising said coating compound or polymer and solvent.

19. The process of claim 18 wherein said solvent is selected from the group consisting of DMF, DMSO, methylene chloride, ethyl acetate, DPMA and mixtures thereof.

20. The process of claim 18 wherein said solvent comprises DMF, DPMA or mixtures thereof.

21. The process of claim 18 wherein said step of reacting the ophthalmic lens with a coating composition comprises a temperature between the freezing and boiling points of said solvent.

22. The process of claim 18 wherein said step of reacting the ophthalmic lens with a coating composition comprises a temperature between 0 and 100°C.

23. The process of claim 18 wherein said step of reacting the ophthalmic lens with a coating composition comprises a temperature between 20 and 50°C.

24. The process of claim 18 wherein said step of reacting the ophthalmic lens with a coating composition comprises a contact time of up to 2 days.

25. The process of claim 18 wherein said step of reacting the ophthalmic lens with a coating composition comprises a contact time of up to 1 day.

26. The process of claim 18 wherein said step of reacting the ophthalmic lens with a coating composition comprises a contact time of up to 12 hours.

27. The process of claim 18 wherein said solution further comprises at least one coupling additive.

28. The process of claim 18 wherein said coupling additive is selected from the group consisting of 4-dimethylaminopyridine (DMAP), 1-(3-dimethylaminopropyl)-3-ethylcarbodiimide hydrochloride salt (EDC), 1,3-diisopropylcarbodiimide, 1,3-dicyclohexylcarbodiimide, 1-hydroxybenzotriazole (HOBt), 1-hydroxybenzotriazole hydrate, crown ethers, acids, bases, enzymes and combinations thereof.

29. An ophthalmic lens formed from curing a reactive monomer mix comprising at least one latent reactive component in an amount effective to provide sufficient bonding sites for reaction with a coating composition, wherein the latent reactive component is at least one ester compound of the formula R-CO-L, wherein R comprises a group capable of cationic, anionic or free radical polymerisation and L is a leaving group.

30. The ophthalmic lens of claim 29 wherein said ophthalmic lens is coated with a coating effective amount of at least one coating compound or polymer comprising one or more nucleophilic moieties selected from the group consisting of alcohol, primary amine, secondary amine, thiol and combinations thereof to form a coated ophthalmic lens.

31. The ophthalmic lens of claim 30 wherein the ophthalmic lens is a contact lens.

32. The ophthalmic lens of claim 30 wherein said reactive monomer mix comprises at least one silicone containing component and at least one hydrophilic component.

**Patentansprüche**

1. Verfahren zum Herstellen einer beschichteten ophthalmischen Linse, umfassend die Schritte:

Härten eines reaktionsfähigen Monomergemischs, um eine ophthalmische Linse zu bilden, wobei das reaktionsfähige Monomergemisch wenigstens eine latent reaktionsfähige Komponente in einer Menge enthält, die wirksam ist, ausreichend Bindungsstellen für die Reaktion mit einer Beschichtungszusammensetzung bereitzustellen, wobei die latent reaktionsfähige Komponente wenigstens eine Esterverbindung der Formel R-CO-L ist, wobei R eine Gruppe umfasst, die zur kationischen, anionischen oder frei-radikalischen Polymerisation fähig ist, und L eine Abgangsgruppe ist, und
Umsetzen der ophthalmischen Linse mit einer Beschichtungszusammensetzung in einer Menge, die ausreicht, um die Oberfläche der ophthalmischen Linse zu bedecken, wobei die Beschichtungszusammensetzung eine oder mehrere nukleophile Einheiten ausgewählt aus der Gruppe bestehend aus Alkohol, primärem Amin, sekundärem Amin, Thiol und Kombinationen davon umfasst, um die beschichtete ophthalmische Linse zu bilden.

2. Verfahren gemäß Anspruch 1, wobei die ophthalmische Linse eine Kontaktlinse ist.

3. Verfahren gemäß Anspruch 1, wobei die R-Gruppe ausgewählt ist aus der Gruppe bestehend aus Acrylaten, Styrylen, Vinylen, $C_{1-6}$Alkylacrylaten, Acrylamiden, $C_{1-6}$Alkylacrylamiden, N-Vinyllactamen, N-Vinylamiden, $C_{2-12}$Alkenylen, $C_{2-12}$Alkenylphenylen, $C_{2-12}$Alkenylnaphthylen, $C_{2-6}$Alkenylphenyl$C_{1-6}$alkylen, Vinylethern und Epoxidgruppen und Kombinationen davon.

4. Verfahren gemäß Anspruch 1, wobei die R-Gruppe ausgewählt ist aus der Gruppe bestehend aus Methacrylaten, Acryloxys, Acrylamiden, Methacrylamiden und Kombinationen davon.

5. Verfahren gemäß Anspruch 1, wobei die L-Gruppe ausgewählt ist aus der Gruppe bestehend aus Hydroxyalkylen, Hydroxyarylen, Hydroxy-paranitroarylen, Alkylestern, Phenylestern, *p*-Nitrophenylestern, N-Hydroxylaminderivativen und Tosylaten, die alle substituiert oder unsubstituiert sein können.

6. Verfahren gemäß Anspruch 1, wobei die L-Gruppe ausgewählt ist aus der Gruppe bestehend aus t-Butylestern, 2,4,5-Trichlorphenylestern, Pentafluorphenylestern, N-Hydroxysuccinimidestern, N-Hydroxyoxodihydrobenzotriazinderivativen und 1-Hydroxybenzotriazolestern.

7. Verfahren gemäß Anspruch 1, wobei die L-Gruppe ausgewählt ist aus der Gruppe bestehend aus Pentafluorphenylestern und N-Hydroxysuccinimidestern.

8. Verfahren gemäß Anspruch 1, wobei die wenigstens eine latent reaktionsfähige Verbindung Pentafluormethacrylat, N-Acryloxysuccinimid und Gemische davon umfasst.

9. Verfahren gemäß Anspruch 1, wobei die latent reaktionsfähige Komponente in dem reaktionsfähigen Monomergemisch in einer Menge zwischen 0,01 und 10 Gew.-% bezogen auf das Gesamtgewicht der reaktionsfähigen Komponenten enthalten ist.

10. Verfahren gemäß Anspruch 1, wobei die latent reaktionsfähige Komponente in dem reaktionsfähigen Monomergemisch in einer Menge zwischen 0,01 und 5 Gew.-% bezogen auf das Gesamtgewicht der reaktionsfähigen Komponenten enthalten ist.

11. Verfahren gemäß Anspruch 1, wobei die latent reaktionsfähige Komponente in dem reaktionsfähigen Monomergemisch in einer Menge zwischen 0,01 und 1 Gew.-% bezogen auf das Gesamtgewicht der reaktionsfähigen Komponenten enthalten ist.

12. Verfahren gemäß Anspruch 2, wobei das reaktionsfähige Monomergemisch wenigstens eine siliconhaltige Komponenten und wenigstens eine hydrophile Komponente umfasst.

13. Verfahren gemäß Anspruch 2, wobei die/das Beschichtungsverbindung oder -polymer ausgewählt ist aus der Gruppe bestehend aus Vitaminen, Antihistaminika, antibakteriellen Mitteln, UV-Blockern, Farbstoffen und Tönungen, biologisch abbaubaren Polymeren, Polyolen, Polyaminen, antimikrobiellen Mitteln, Netzmitteln, Metallchelatbildnern, Lachrymatoren, Prodrugs, Peptidoglycanen, Oligosacchariden, Polysacchariden, Aminoglycosiden, Glycopeptiden und Kombinationen davon.

14. Verfahren gemäß Anspruch 2, wobei die/das Beschichtungsverbindung oder -polymer ausgewählt ist aus der Gruppe bestehend aus polyHEMA, β-Lactam-Antibiotika, die mit einer Aminogruppe oder einer Hydroxygruppe funktionalisiert sind, Penicillinen, die mit einer Aminogruppe oder einer Hydroxygruppe funktionalisiert sind, Phenylglycin, 4-Hydroxyphenylgycin, Cephalosporinen, die mit einer Aminogruppe oder einer Hydroxygruppe funktionalisiert sind, Cephaloglycin, Cephalexin, Cephadroxil, Carbapenemen, die mit einer Aminogruppe oder einer Hydroxygruppe funktionalisiert sind, Streptomycin, Gentomicin, Amikacin, Oxazolidinonen, die mit einer Aminogruppe oder einer Hydroxygruppe funktionalisiert sind, Tetracyclinen, die mit einer Aminogruppe oder einer Hydroxygruppe funktionalisiert sind, Glycylcyclinen, die mit einer Aminogruppe oder einer Hydroxygruppe funktionalisiert sind, Chinolonen, die mit einer Aminogruppe oder einer Hydroxygruppe funktionalisiert sind, Fluorchinolonen, die mit einer Aminogruppe oder einer Hydroxygruppe funktionalisiert sind, Macroliden, die mit einer Aminogruppe oder einer Hydroxygruppe funktionalisiert sind, Ketoliden, die mit einer Aminogruppe oder einer Hydroxygruppe funktionalisiert sind, Streptograminen, die mit einer Aminogruppe oder einer Hydroxygruppe funktionalisiert sind, Vancomycinderivaten, die mit einer Aminogruppe oder einer Hydroxygruppe funktionalisiert sind, Teicoplaninderivaten, die mit einer Aminogruppe oder einer Hydroxygruppe funktionalisiert sind, Avoparcinderivaten, die mit einer Aminogruppe oder einer Hydroxygruppe funktionalisiert sind, und Kombinationen davon.

15. Verfahren gemäß Anspruch 2, wobei die/das Beschichtungsverbindung oder -polymer ausgewählt ist aus der Gruppe bestehend aus Netzmitteln, antimikrobiellen Mitteln, UV-Blockern, antibakteriellen Mitteln, biologisch abbaubaren Polymeren, Kombinationen davon.

16. Verfahren gemäß Anspruch 2, wobei die/das Beschichtungsverbindung oder -polymer ein Polymer ausgewählt aus der Gruppe bestehend aus Polyalkoholen, Polyaminen, bioaktiven Verbindungen, die Amin- und/oder Alkoholfunktionalitäten umfassen, und Gemischen davon ist.

17. Verfahren gemäß Anspruch 2, wobei die/das Beschichtungsverbindung oder -polymer polyHEMA und Gemische davon umfasst.

18. Verfahren gemäß Anspruch 1, wobei der Schritt des Umsetzens der ophthalmischen Linse mit einer Beschichtungszusammensetzung Platzieren der ophthalmischen Linse in einer Lösung umfasst, die die/das Beschichtungsverbindung oder -polymer und ein Lösungsmittel umfasst.

19. Verfahren gemäß Anspruch 18, wobei das Lösungsmittel ausgewählt ist aus der Gruppe bestehend aus DMF, DMSO, Methylenchlorid, Ethylacetat, DPMA und Gemischen davon.

20. Verfahren gemäß Anspruch 18, wobei das Lösungsmittel DMF, DPMA oder Gemische davon umfasst.

21. Verfahren gemäß Anspruch 18, wobei der Schritt des Umsetzens der ophthalmischen Linse mit einer Beschichtungszusammensetzung eine Temperatur zwischen dem Gefrier- und dem Siedepunkt des Lösungsmittels umfasst.

22. Verfahren gemäß Anspruch 18, wobei der Schritt des Umsetzens der ophthalmischen Linse mit einer Beschichtungszusammensetzung eine Temperatur zwischen 0 und 100 °C umfasst.

23. Verfahren gemäß Anspruch 18, wobei der Schritt des Umsetzens der ophthalmischen Linse mit einer Beschichtungszusammensetzung eine Temperatur zwischen 20 und 50 °C umfasst.

24. Verfahren gemäß Anspruch 18, wobei der Schritt des Umsetzens der ophthalmischen Linse mit einer Beschichtungszusammensetzung eine Kontaktzeit bis zu 2 Tagen umfasst.

25. Verfahren gemäß Anspruch 18, wobei der Schritt des Umsetzens der ophthalmischen Linse mit einer Beschichtungszusammensetzung eine Kontaktzeit bis zu 1 Tag umfasst.

26. Verfahren gemäß Anspruch 18, wobei der Schritt des Umsetzens der ophthalmischen Linse mit einer Beschichtungszusammensetzung eine Kontaktzeit bis zu 12 Stunden umfasst.

27. Verfahren gemäß Anspruch 18, wobei die Lösung ferner wenigstens einen Kopplungs-Zusatzstoff umfasst.

28. Verfahren gemäß Anspruch 18, wobei der Kopplungs-Zusatzstoff ausgewählt ist aus der Gruppe bestehend aus 4-Dimethylaminopyridin (DMAP), 1-(3-Dimethylaminopropyl)-3-ethylcarbodiimidhydrochloridsalz (EDC), 1,3-Diisopropylcarbodiimid, 1,3-Dicyclohexylcarbodiimid, 1-Hydroxybenzotriazol (HOBt), 1-Hydroxybenzotriazolhydrat, Kronenethern, Säuren, Basen, Enzymen und Kombinationen davon.

29. Ophthalmische Linse, gebildet durch Härten eines reaktionsfähigen Monomergemischs, das wenigstens eine latent reaktionsfähige Komponente in einer Menge enthält, die wirksam ist, ausreichend Bindungsstellen für die Reaktion mit einer Beschichtungszusammensetzung bereitzustellen, wobei die latent reaktionsfähige Komponente wenigstens eine Esterverbindung der Formel R-CO-L ist, wobei R eine Gruppe umfasst, die zur kationischen, anionischen oder frei-radikalischen Polymerisation fähig ist, und L eine Abgangsgruppe ist.

**30.** Ophthalmische Linse gemäß Anspruch 29, wobei die ophthalmische Linse mit einer beschichtungswirksamen Menge von wenigstens einer/einem Beschichtungsverbindung oder -polymer beschichtet ist, die/das eine oder mehrere nukleophile Einheiten ausgewählt aus der Gruppe bestehend aus Alkohol, primärem Amin, sekundärem Amin, Thiol und Kombinationen davon umfasst, um eine beschichtete ophthalmische Linse zu bilden.

**31.** Ophthalmische Linse gemäß Anspruch 30, wobei die ophthalmische Linse eine Kontaktlinse ist.

**32.** Ophthalmische Linse gemäß Anspruch 30, wobei das reaktionsfähige Monomergemisch wenigstens eine siliconhaltige Komponente und wenigstens eine hydrophile Komponente umfasst.

**Revendications**

**1.** Procédé de fabrication d'une lentille ophtalmique revêtue comprenant les étapes de :

durcissement d'un mélange de monomères réactifs pour former une lentille ophtalmique, le mélange de monomères réactifs comprenant au moins un composant réactif latent en une quantité efficace pour fournir des sites de liaison suffisants pour réaction avec une composition de revêtement, dans lequel le composant réactif latent est au moins un composé ester de formule R-CO-L, dans laquelle R comprend un groupe capable de polymérisation cationique, anionique ou radicalaire et L est un groupe partant, et
réaction de la lentille ophtalmique avec une composition de revêtement en une quantité suffisante pour revêtir la surface de la lentille ophtalmique, la composition de revêtement comprenant un ou plusieurs fragments nucléophiles choisis dans le groupe constitué d'un alcool, une amine primaire, une amine secondaire, un thiol et des combinaisons de ceux-ci pour former la lentille ophtalmique revêtue.

**2.** Procédé de la revendication 1 dans lequel la lentille ophtalmique est une lentille de contact.

**3.** Procédé de la revendication 1 dans lequel ledit groupe R est choisi dans le groupe constitué des acrylates, styryles, vinyles, acrylates d'alkyle en $C_{1-6}$, acrylamides, (alkyle en $C_{1-6}$)acrylamides, N-vinyllactames, N-vinylamides, alcényles en $C_{2-12}$, (alcényle en $C_{2-12}$) phényles, (alcényle en $C_{2-12}$) naphtyles, (alcényle en $C_{2-6}$) phényl (alkyles en $C_{1-6}$), éthers vinyliques et groupes époxyde et des combinaisons de ceux-ci.

**4.** Procédé de la revendication 1 dans lequel ledit groupe R est choisi dans le groupe constitué de méthacrylates, acryloxys, acrylamides, méthacrylamides et des combinaisons de ceux-ci.

**5.** Procédé de la revendication 1 dans lequel ledit groupe L est choisi dans le groupe constitué d'hydroxyalkyles, hydroxyaryles, hydroxy-paranitroaryles, esters d'alkyle, esters de phényle, esters de p-nitrophényle, dérivés de N-hydroxylamine et tosylates, tous pouvant être substitués ou non substitués.

**6.** Procédé de la revendication 1 dans lequel ledit groupe L est choisi dans le groupe constitué d'esters de *t*-butyle, esters de 2,4,5-trichlorophényle, esters de pentafluorophényle, esters de N-hydroxysuccinimide, dérivés de N-hydroxy-oxo-dihydrobenzotriazine et esters de 1-hydroxybenzotriazole.

**7.** Procédé de la revendication 1 dans lequel ledit groupe L est choisi dans le groupe constitué d'esters de pentafluorophényle et esters de N-hydroxysuccinimide.

**8.** Procédé de la revendication 1 dans lequel ledit au moins un composé réactif latent comprend le pentafluorométhacrylate, le N-acryloxysuccinimide et des mélanges de ceux-ci.

**9.** Procédé de la revendication 1 dans lequel ledit composant réactif latent est inclus dans le mélange de monomères réactifs en une quantité comprise entre 0,01 et 10 % en poids sur la base du poids total des composants réactifs.

**10.** Procédé de la revendication 1 dans lequel ledit composant réactif latent est inclus dans le mélange de monomères réactifs en une quantité comprise entre 0,01 et 5 % en poids sur la base du poids total des composants réactifs.

**11.** Procédé de la revendication 1 dans lequel ledit composant réactif latent est inclus dans le mélange de monomères réactifs en une quantité comprise entre 0,01 et 1 % en poids sur la base du poids total des composants réactifs.

12. Procédé de la revendication 2 dans lequel ledit mélange de monomères réactifs comprend au moins un composant contenant de la silicone et au moins un composant hydrophile.

13. Procédé de la revendication 2 dans lequel ledit composé ou polymère de revêtement est choisi dans le groupe constitué de vitamines, antihistaminiques, antibactériens, anti-UV, colorants et teintures, polymères biodégradables, polyols, polyamines, antimicrobiens, agents mouillants, chélateurs de métaux, substances lacrymogènes, promédicaments, peptidoglycanes, oligosaccharides, polysaccharides, aminoglycosides, glycopeptides et des combinaisons de ceux-ci.

14. Procédé de la revendication 2 dans lequel ledit composé ou polymère de revêtement est choisi dans le groupe constitué de polyHEMA, antibiotiques β-lactames fonctionnalisés avec l'un parmi un groupe amino ou un groupe hydroxyle, pénicillines fonctionnalisées avec l'un parmi un groupe amino ou un groupe hydroxyle, phénylglycine, 4-hydroxyphénylgycine, céphalosporines fonctionnalisées avec l'un parmi un groupe amino ou un groupe hydroxyle, céphaloglycine, céphalexine, céphadroxil, carbapénèmes fonctionnalisés avec l'un parmi un groupe amino ou un groupe hydroxyle, streptomycine, gentamicine, amikacine, oxazolidinones fonctionnalisées avec l'un parmi un groupe amino ou un groupe hydroxyle, tétracyclines fonctionnalisées avec l'un parmi un groupe amino ou un groupe hydroxyle, glycylcyclines fonctionnalisées avec l'un parmi un groupe amino ou un groupe hydroxyle, quinolones fonctionnalisées avec l'un parmi un groupe amino ou un groupe hydroxyle, fluoroquinolones fonctionnalisées avec l'un parmi un groupe amino ou un groupe hydroxyle, macrolides fonctionnalisés avec l'un parmi un groupe amino ou un groupe hydroxyle, cétolides fonctionnalisés avec l'un parmi un groupe amino ou un groupe hydroxyle, streptogramines fonctionnalisées avec l'un parmi un groupe amino ou un groupe hydroxyle, dérivés de vancomycine fonctionnalisés avec l'un parmi un groupe amino ou un groupe hydroxyle, dérivés de teïcoplanine fonctionnalisés avec l'un parmi un groupe amino ou un groupe hydroxyle, dérivés d'avoparcine fonctionnalisés avec l'un parmi un groupe amino ou un groupe hydroxyle et des combinaisons de ceux-ci.

15. Procédé de la revendication 2 dans lequel ledit composé ou polymère de revêtement est choisi dans le groupe constitué d'agents mouillants, antimicrobiens, anti-UV, antibactériens, polymères biodégradables, et des combinaisons de ceux-ci.

16. Procédé de la revendication 2 dans lequel ledit composé ou polymère de revêtement est un polymère choisi dans le groupe constitué de polyalcools, polyamines, composés bioactifs comprenant des fonctionnalités amine et/ou alcool et des mélanges de ceux-ci.

17. Procédé de la revendication 2 dans lequel ledit composé ou polymère de revêtement comprend polyHEMA, et des mélanges de ceux-ci.

18. Procédé de la revendication 1 dans lequel ladite étape de réaction de la lentille ophtalmique avec une composition de revêtement comprend le placement de ladite lentille ophtalmique dans une solution comprenant ledit composé ou polymère de revêtement et un solvant.

19. Procédé de la revendication 18 dans lequel ledit solvant est choisi dans le groupe constitué de DMF, DMSO, dichlorométhane, acétate d'éthyle, DPMA et des mélanges de ceux-ci.

20. Procédé de la revendication 18 dans lequel ledit solvant comprend DMF, DPMA ou des mélanges de ceux-ci.

21. Procédé de la revendication 18 dans lequel ladite étape de réaction de la lentille ophtalmique avec une composition de revêtement comprend une température entre les points de congélation et d'ébullition dudit solvant.

22. Procédé de la revendication 18 dans lequel ladite étape de réaction de la lentille ophtalmique avec une composition de revêtement comprend une température comprise entre 0 et 100 °C.

23. Procédé de la revendication 18 dans lequel ladite étape de réaction de la lentille ophtalmique avec une composition de revêtement comprend une température comprise entre 20 et 50 °C.

24. Procédé de la revendication 18 dans lequel ladite étape de réaction de la lentille ophtalmique avec une composition de revêtement comprend un temps de contact allant jusqu'à 2 jours.

25. Procédé de la revendication 18 dans lequel ladite étape de réaction de la lentille ophtalmique avec une composition de revêtement comprend un temps de contact allant jusqu'à 1 jour.

26. Procédé de la revendication 18 dans lequel ladite étape de réaction de la lentille ophtalmique avec une composition de revêtement comprend un temps de contact allant jusqu'à 12 heures.

27. Procédé de la revendication 18 dans lequel ladite solution comprend en outre au moins un additif de couplage.

28. Procédé de la revendication 18 dans lequel ledit additif de couplage est choisi dans le groupe constitué des 4-diméthylaminopyridine (DMAP), sel de chlorhydrate de 1-(3-diméthylaminopropyl)-3-éthylcarbodiimide (EDC), 1,3-diisopropylcarbodiimide, 1,3-dicyclohexylcarbodiimide, 1-hydroxybenzotriazole (HOBt), 1-hydroxybenzotriazole hydraté, éthers couronnes, acides, bases, enzymes et des combinaisons de ceux-ci.

29. Lentille ophtalmique formée par durcissement d'un mélange de monomères réactifs comprenant au moins un composant réactif latent en une quantité efficace pour fournir des sites de liaison suffisants pour réaction avec une composition de revêtement, où le composant réactif latent est au moins un composé ester de formule R-CO-L, dans laquelle R comprend un groupe capable de polymérisation cationique, anionique ou radicalaire et L est un groupe partant.

30. Lentille ophtalmique de la revendication 29, ladite lentille ophtalmique étant revêtue avec une quantité efficace de revêtement d'au moins un composé ou polymère de revêtement comprenant un ou plusieurs fragments nucléophiles choisis dans le groupe constitué d'un alcool, une amine primaire, une amine secondaire, un thiol et des combinaisons de ceux-ci pour former une lentille ophtalmique revêtue.

31. Lentille ophtalmique de la revendication 30, la lentille ophtalmique étant une lentille de contact.

32. Lentille ophtalmique de la revendication 30, ledit mélange de monomères réactifs comprenant au moins un composant contenant une silicone et au moins un composant hydrophile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5741551 A
- US 5002582 A
- US 6087415 A
- US 4711943 A
- US 5070215 A
- US 6020445 A
- US 5760100 A
- US 6367929 B
- US 5034461 A
- US 5484863 A
- US 5690953 A
- US 5304584 A
- US 5565539 A
- US 4910277 A
- US 6020455 A
- US 3408429 A
- US 3660545 A
- US 4113224 A
- US 4197266 A
- US 4495313 A
- US 4680336 A
- US 4889664 A
- US 5039459 A